(19) Europäisches Patentamt
European Patent Office
Office européen des brevets

(11) Publication number: **0 007 766**
**B1**

(12) **EUROPEAN PATENT SPECIFICATION**

(45) Date of publication of patent specification: **28.12.83**

(51) Int. Cl.³: **C 07 J 13/00**, C 07 J 21/00

(21) Application number: **79301411.9**

(22) Date of filing: **17.07.79**

(54) **Epoxypregnadienes, hydroxypregnatrienes, process for their preparation and process for their conversion to precursors of useful anti-inflammatory agents.**

(30) Priority: **31.07.78 US 929508**

(43) Date of publication of application:
**06.02.80 Bulletin 80/3**

(45) Publication of the grant of the patent:
**28.12.83 Bulletin 83/52**

(84) Designated Contracting States:
**CH DE FR GB IT NL**

(56) References cited:
**DE - B - 1 301 999**
**US - A - 2 873 272**
**US - A - 3 056 807**
**US - A - 3 068 251**

(73) Proprietor: **THE UPJOHN COMPANY**
**301 Henrietta Street**
**Kalamazoo, Michigan 49001 (US)**

(72) Inventor: **Shephard, Kenneth Paul**
**2722 Kalarama**
**Portage Michigan (US)**

(74) Representative: **Perry, Robert Edward et al,**
**GILL JENNINGS & EVERY 53-64 Chancery Lane**
**London WC2A 1HN (GB)**

**EP 0 007 766 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Epoxypregnadienes, hydroxypregnatrienes, process for their preparation and process for their conversion to precursors of useful anti-inflammatory agents

This invention relates to novel 17α,20α-epoxypregna-1,4-dien-3-one 21-acylates (I), their conversion to 21-hydroxypregna-1,4,16-trien-3-one 21-acylates (II), their preparation from 21-hydroxypregna-1,4,17(20)-trien-3-one 21-acylates (O), and the preparation of 20,21-dihydroxypregna-1,4,16-trien-3-ones (III) by hydrolysis of the formula II compounds. The formula III compounds are useful as intermediates in the production of anti-inflammatory agents.

It will be appreciated that the preparation of the formula II compounds from the formula O compounds *via* the formula I epoxides involves a change in position of a carbon-carbon double bond.

U.S. Patent Specification No 3,444,216 discloses 11β,21-dihydroxypregna-4-en-3-one. U.S. Patent Specification No 3,375,261 discloses 21-acylated steroids having a $\Delta^1$double bond.

The reaction of a ketone with a peracid is well known in the art and has been termed the Baeyer-Villiger reaction (the oxidation of ketones with peracids). In the Baeyer-Villiger reaction, an oxygen atom is inserted between the carbonyl group and the alkyl portion of the ketone, thereby forming an ester. In the case of a cyclic ketone, such as a 3-keto steroid, it is well known that the resulting product is the corresponding A-ring lactone, as is shown in Organic Reactions 9, 73 (1957) and J. Med. Pharm. Chem. 5, 714 (1962). Page 79 of the former reference shows the exact problem that may be anticipated when epoxidising a carbon-carbon double bond in a 3-keto steroid while wishing not to affect the A-ring. Page 715 of the latter reference discloses the Baeyer-Villiger reaction when the reactant is a $\Delta^4$-3-keto steroid. The lactone and subsequent side reaction products are formed. It might therefore be anticipated that a not insignificant amount of Baeyer-Villiger product (lactone) would be obtained when any 3-keto steroid or, more specifically, when a $\Delta^4$-3-keto or a $\Delta^{1,4}$-3-keto steroid was reacted with a peracid.

In contrast, the U.S. Patent Specification No. 3,876,633 discloses oxidation of a $\Delta^{17(20)}$-steroid to the corresponding 17α,20α-epoxy-steroid in a 97% yield (weight). This is not unexpected because the steroid used in this process did not have a ketone group in ring A and therefore no side reaction (Baeyer-Villiger reaction) was possible.

Surprisingly it has been found that, when a compound of formula O is reacted with a peracid, a formula I compound is formed and the amount of any Baeyer-Villiger reaction products can be very low.

It is known that the position of carbon-carbon double bonds in steroids can be changed. For example, U.S. Patent Specification No. 3,725,392 discloses a process for producing various 9α-fluoro-16β-alkylprednisolones, including 9α-fluoro-16β-methylprednisolone (betamethasone), which are known to be highly active anti-inflammatory agents. The prednisolones type steroids have a $\Delta^{1,4}$-3-keto A ring. This specification discloses that the process can be performed on a prednisolone-type compound or on a cortisone-type ($\Delta^4$-3-keto) compound, the $\Delta^1$ double bond being put in later. The first 2 steps of the disclosed process comprise the transformation of a 11β,21-dihydroxypregna-4,17(20)-dien-3-one to a 11β,20,21-trihydroxypregna-4,16-dien-3-one, using photosensitised oxygenation followed by reduction. Example 1 of this specification discloses that, starting with 165.2 g (0.5 mole) of the $\Delta^{17(20)}$ compound, 51.1 g (0.15 moles) of the $\Delta^{16}$-21-ol are obtained, a 30% chemical yield.

U.S. Patent Specification No. 3,281,415 discloses the transformation of a 17(20)-olefinic steroid to the corresponding $\Delta^{16}$-20-keto steroid. Claim 20 claims a process of producing compounds of formulae II and III of the present invention using photosensitised oxygenation. In column 30, at line 8, 11β,20α,21-trihydroxy-1,4,16-pregnatrien-3-one 21-acetate is disclosed as a starting material for the preparation of the corresponding 20-keto compound, but there is no indication of how the starting material might be prepared.

German Specification No. 1,301,999 discloses a process for transforming a 17α,20α-epoxypregnane to a $\Delta^{16}$-21-ol in about 90% yield by use of 1.82 equivalents of hydrogen bromide. The 10% impurity is the 17β-bromide.

German Specification No. 1,297,604 discloses a process for transforming a $\Delta^{17(20)}$-pregnane into a $\Delta^{16}$-21-ol by peracid oxidation of the Δ17(20)-pregnane to the corresponding 17α,20α-epoxide which is then reacted with hydrogen iodide to form a 17β-iodide which is then transformed to the $\Delta^{16}$-21-ol.

U.S. Patent Specification No. 4,089,852 discloses the preparation of 21-hydroxypregn-16-3n-20-ones by alkylating a 17-keto steroid with a lithium reagent of the formula $LiC(OR_4){=}CHOR_3$, thereby introducing, at the 17-position, a β-hydroxy group and a $-C(OR_4){=}CHOR_3$ group in the α-position, eliminating the 17β-hydroxy and hydrolysing the $OR_4$ group. It is to be noted that this process does not involve the step-wise migration of a carbon-carbon double bond. Further, this process requires an expensive lithium reagent. The claims are limited to the use of 17-keto steroids which do not have any other free keto function and the reaction is therefore apparently inapplicable to 3-ketopregnanes.

Surprisingly, it has been found that a formula I compound can be reacted to form a formula II compound in good yield, using only a low amount of a hydrogen halide and producing only a small amount of the 17β-halide, apparently without forming that compound as an intermediate.

Compounds of formula II are disclosed in U.S. Patent Specifications Nos. 3,068,251 and

3,056,807. Both these specifications disclose 11$\beta$,20$\beta$,21-trihydroxy-1,14,16-pregnatrien-3-one 21-acetate.

Novel compounds according to the present invention are of formula I:

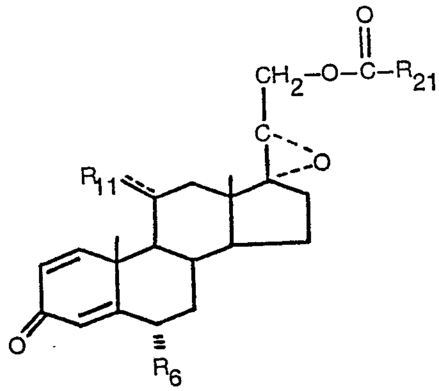

I

wherein $R_6$ is hydrogen, methyl or fluorine; $R_{11}$ is $\beta$-hydroxy or oxo; and $R_{21}$ is $C_{1-3}$ alkyl or phenyl optionally substituted by up to 3 substituents independently selected from fluorine, chlorine, methyl, methoxy and nitro.

According to a first process of the invention, a compound of formula I as defined above is prepared by oxidising an olefin of formula O:

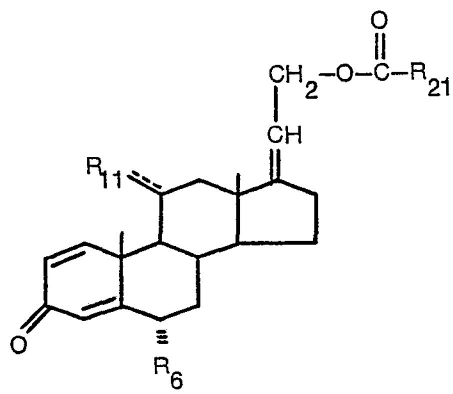

O

wherein $R_6$, $R_{11}$ and $R_{21}$ are as defined above, with at least one molar equivalent of a peracid.

According to a second process of the invention, a compound of formula II:

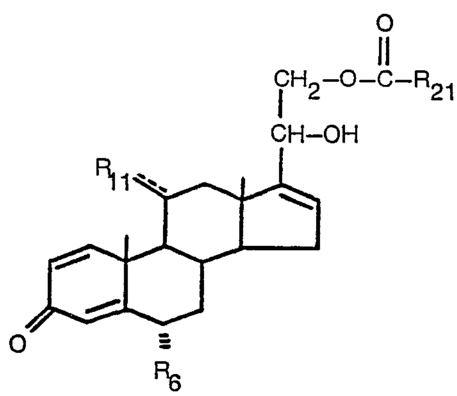

II

wherein $R_6$, $R_{11}$ and $R_{21}$ are as defined above, is prepared by reacting an epoxide of formula I as defined above with hydrochloric or hydrobromic acid.

According to a third process of the invention, a compound of formula III:

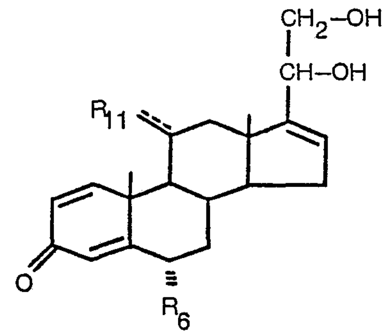

III

wherein $R_6$ and $R_{11}$ are as defined above, is prepared by base hydrolysis of a compound of formula II as defined above.

In the formulae in this specification, the ⎓ bond by which the $R_{11}$ substituent is attached to the C ring of the steroids means that there is a single bond when $R_{11}$ is β-hydroxy and a double bond when $R_{11}$ is oxo.

The peracid oxidation of the 17(20) olefin of formula O to the 17α,20α-epoxide of formula I can be performed with virtually any peracid. The preferred peracids are peracetic, perpropionic, perbenzoic and *m*-chloroperbenzoic acid. The most preferred peracid is peracetic acid. Peracids (especially peracetic acid) are commercially available or may be readily prepared from the corresponding acid by known procedures. At least 1.0 equivalent of the peracid is required. Usually no more than 5.0 equivalents are used and it is preferred that about 1.5 equivalents of the peracid are used. The peracid oxidation can be performed in most non-polar solvents, such as methylene chloride, chloroform, toluene, cyclohexane and Skellysolve B (a mixture of isomeric hexanes). The reaction can be performed at a temperature of about 0°C to the reflux temperature of the particular solvent utilised. It is preferred that the reaction temperature is 20—25°C. At colder temperatures, the reaction proceeds slowly and, while the reaction proceeds faster at high temperatures, heating is required. At 20—25°C the reaction is complete after stirring overnight. When the oxidation reaction is complete as measured by thin layer chromatography, any excess peracid is destroyed by an inorganic reducing agent such as sodium thiosulphate or sodium sulphite.

The epoxide of formula I is opened by reaction with either hydrochloric or, preferably, hydrobromic acid. The amount of the acid is usually below 0.5 equivalents; the preferred amount is 0.03—0.05 equivalents. More than 0.05 equivalents of acid can be used but, the greater the amount of acid, the greater the likelihood of hydrolysis of the $C_{21}$ ester and formation of the 17β-halide. The epoxide opening is best performed in polar oxygenated solvents such as tetrahydrofuran (THF), dioxane or diethyl ether; THF is the preferred solvent. It is preferred that the polar oxygenated solvent is dry as less acid is then required. This reaction is preferably performed at 20—25°C although lower or higher temperatures will also work. The acid is best added slowly to the epoxide, in order to avoid side reactions.

The hydrolysis of the formula II compound is performed utilising a strong base such as sodium hydroxide, potassium hydroxide, a methoxide, an ethoxide, sodium carbonate, potassium carbonate, sodium bicarbonate or potassium bicarbonate. The hydrolysis reaction is performed in a polar solvent such as acetone and/or alcohol. At 20—25°C the reaction is complete in about 30 minutes. For weaker bases the reaction time will be a little longer.

In the present invention, $R_6$ is preferably hydrogen, $R_{11}$ is preferably β-hydroxy and $R_{21}$ is preferably methyl. The invention is particularly suitable for the preparation of 11β,20,21-trihydroxypregna-1,4,16-trien-3-one (III) via 11β,20,21-trihydroxypregna-1,4,16-trien-3-one 21-acetate (II), 11β,21-dihydroxy-17α,20α-epoxypregna-1,4-dien-3-one 21-acetate (I) and 11β,21-dihydroxypregna-1,4,17(20)-trien-3-one 21-acetate (O). The preferred compound of formula III is known from U.S. Patent Specification No. 3,725,392. Therefore the preferred compound of formula (III) is an important intermediate in the synthesis of 9α-fluoro-16β-methylprednisolone (betamethasone) which is an important anti-inflammatory steroid. The compounds of formulae I and II in which $R_6$ is methyl and $R_{11}$ is β-hydroxy are therefore important intermediates in the synthesis of betamethasone.

The following Examples illustrate the invention. Comparative Examples are provided for reference. In the Examples, TLC refers to thin-layer chromatography; THF refers to tetrahydrofuran; SSB refers to a mixture of isomeric hexanes; "saline" refers to an aqueous saturated sodium chloride solution; $[\alpha]_D^{25}$ refers to the angle of rotation of plane polarised light (specific optical rotation) at 25°C with the sodium D line (5893 A).

Example 1

11β,21-Dihydroxy-17α,20α-epoxypregna-1,4-dien-3-one 21-acetate (I)

79.5 g of 11β,21-dihydroxypregna-1,4,17(20)-trien-3-one 21-acetate (formula O — see U.S. Patent Specification No. 2,842,569), 800 ml of methylene chloride and 9.3 g of anhydrous sodium acetate are stirred under nitrogen at 19°C. Peracetic acid (40%, 55 ml) is added to the mixture over a

period of 10 minutes. The reaction mixture is stirred for 20 hours at 20—25°C. Sodium thiosulfate solution (15%, 200 ml) is added to destroy the excess peracetic acid. The mixture is then washed with aqueous sodium bicarbonate solution (6%, 600 ml) and a half-saturated sodium chloride solution (250 ml). The mixture is dried over magnesium sulfate and concentrated on a steam bath at 80°C at atmospheric pressure while replacing the methylene chloride with SSB. The mixture is concentrated to 250 ml, SSB (500 ml) is added, the mixture is concentrated to 425 ml, SSB (300 ml) is added and the mixture is concentrated to 425 ml at 65°C. The mixture is then cooled to 5°C and filtered. The solids are washed with SSB and dried under reduced pressure at 70°C to give the title compound, 81.86 g (98.7% chemical yield), m.p. 174-185°C; $[\alpha]_D^{25}$ + 81.8° (chloroform).

## Example 2

11β,21-Dihydroxy-17α,20α-epoxypregna-1,4-dien-3-one 21-acetate (I)

11β,21-Dihydroxypregna-1,4,17(20)-trien-3-one 21-acetate (1 kg), sodium acetate (117 g) and methylene chloride (10 l) are stirred at 20°C. Peracetic acid (40% 692 ml) is added over a period of an hour and the mixture is stirred overnight at 20°C. The reaction mixture is washed with anhydrous sodium thiosulfate (15%, 2.83 l). The organic phase is then washed with aqueous sodium bicarbonate (9%, 6.5 l) followed by a wash with an aqueous sodium chloride solution (13%, 3.1 l). The methylene chloride is then replaced by SSB by the following procedure: the organic phase is concentrated at atmospheric pressure to about 3.1 l, SSB (4.3 l) is added, the mixture is again concentrated at atmospheric pressure to about 5.3 l, SSB (2.6 l) is added and the mixture once again concentrated to about 5.3 l. The mixture is then cooled and filtered to obtain the title compound, 1.03 kg (98.7% chemical yield).

## Comparative Example A

A solution of 11β,21-dihydroxypregna-4,17(20)-dien-3-one 21-acetate (42.0 g) in methylene chloride (450 ml) is stirred with cooling at 0°C while a mixture of *m*-chloroperbenzoic acid (26.4 g, 80.2% pure) is added over a period of 13 minutes. The mixture is stirred at 0—5°C for 90 minutes and then at 20—25°C for 16 hours. The reaction mixture is washed with a sodium bicarbonate solution (5%) and then with water. The methylene chloride solution is dried over anhydrous magnesium sulfate and the organic diluent is removed under reduced pressure while heating in a water bath at 50—60°C. The residue is crystallised from acetone to give 17α,20α-epoxy-11β,21-dihydroxypregna-4-en-3-one 21-acetate (34.314 g). A second crop of crystals was obtained (3.0 g) containing about 50% of the desired epoxide.

The total yield is about 35.81 g or 81.8%. The TLC (ethyl acetate/benzene; 45/55 v/v) of the mother liquid shows a substantial amount of a Baeyer-Villiger by-product as might be expected on the basis of what is known about reaction of ketones ($\Delta^4$-3-keto function of ring A) with peracids.

## Example 3

11β,20,21-Trihydroxypregna-1,4,16-trien-3-one 21-acetate (II)

11β,21-Dihydroxy-17α,20α-epoxypregna-1,4-dien-3-one 21-acetate (Example 1, 20 g) in THF (700 ml) is stirred at 23.5°C. A solution of hydrogen bromide (48%, 0.31 ml) in THF (100 ml) is added to the mixture over a period of 38 minutes. The reaction temperature increases from 23.5° to 25.5°C during the hydrogen bromide addition. The mixture is stirred for 20 minutes and then concentrated under reduced pressure at 43°C to an oil. The oil is dissolved in methylene chloride (50 ml) at reflux. The mixture is concentrated on a steam bath at atmospheric pressure while replacing the methylene chloride with diethyl ether. When all the methylene chloride is removed the mixture is stirred at 20—25°C for 3 hours and filtered. The solids are washed with diethyl ether and dried under reduced pressure at 70°C to give the title compound, 18.1 g (90.5% chemical yield), m.p. 121.5—135.5°C; $[\alpha]_D^{25}$ + 56.3° (chloroform).

## Example 4

11β,20,21-Trihydroxypregna-1,4,16-trien-3-one 21-acetate (II)

11β,21-Dihydroxy-17α,20α-epoxypregna-1,4-dien-3-one 21-acetate (Example 2, 1.0 kg) in THF (33 kg) is stirred at 25°C. Hydrogen bromide (48%, 15 ml) in THF (4.4 l) is added over a period of one hour and the mixture is stirred for another hour at 25—30°C. The mixture is concentrated under reduced pressure to 2 l, acetone (6.3 kg) is added and the mixture is kept at 20—25°C. The title compound may be isolated, if so desired, by the procedure of Example 3.

## Comparative Example B

Following the procedures of Examples 1 and 3, 11β,21-dihydroxypregna-1,4-17(20)-trien-3-one, i.e. the non-acylated compound, was converted to 11β,20,21-trihydroxypregna-1,4,16-trien-3-one, compounds disclosed in U.S. Patent Specification No. 3,725,392, at temperatures of 15°C and 30°C (23.5—25.5°C was used in Example 3 above). At 15°C the overall chemical yield was 49.5% and was 74.2% at 30°C. While the yield is clearly temperature dependent, the overall chemical yield of Example 1 and 3 above was about 89%.

Example 5

11$\beta$,20,21-Trihydroxypregna-1,4,16-trien-3-one (III)

11$\beta$,20,21-Trihydroxypregna-1,4,16-trien-3-one 21-acetate (Example 4, 16.5 g) is dissolved in acetone (170 ml) and stirred at 20—25°C. Methanol (10 ml) and sodium methoxide in methanol (25%, 1.25 ml) are added and the mixture is stirred for 30 minutes. Acetic acid is added to neutralise the base, the mixture is concentrated under reduced pressure with a little heat and the title compound is obtained by crystallisation.

Example 6

11$\beta$,20,21-Trihydroxypregna-1,4,16-trien-3-one (III)

Methanol (0.5 l) and sodium methoxide (25% in methanol, 75 ml) are added to 11$\beta$,20,21-trihydroxypregna-1,4,16-trien-3-one 21-acetate (Example 3) in acetone and the mixture is stirred for 30 minutes at 20—25°C. The title compound is obtained by the work-up procedure of Example 5.

**Claims**

1. A compound of formula I:

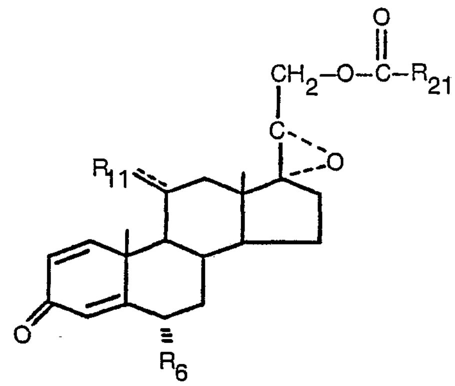

I

wherein $R_6$ is hydrogen, methyl or fluorine; $R_{11}$ is $\beta$-hydroxy or oxo; and $R_{21}$ is $C_{1-3}$ alkyl or phenyl optionally substituted by up to 3 substituents independently selected from fluorine, chlorine, methyl, methoxy and nitro.

2. 11$\beta$21-Dihydroxy-17$\alpha$,20$\alpha$-epoxypregna-1,4-dien-3-one 21-acetate.

3. A process for preparing a compound as claimed in claim 1 or claim 2, which comprises oxidising an olefin of formula O;

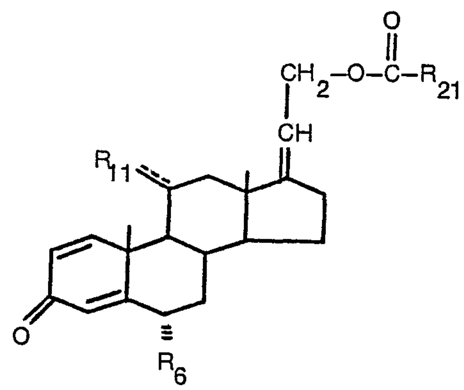

O

wherein $R_6$, $R_{11}$ and $R_{21}$ are as defined in claim 1, with at least one molar equivalent of a peracid.

4. A process according to claim 3, wherein the peracid is selected from peracetic acid, perpropionic acid, perbenzoic acid and *m*-chloroperbenzoic acid.

5. A process for preparing a compound of formula II:

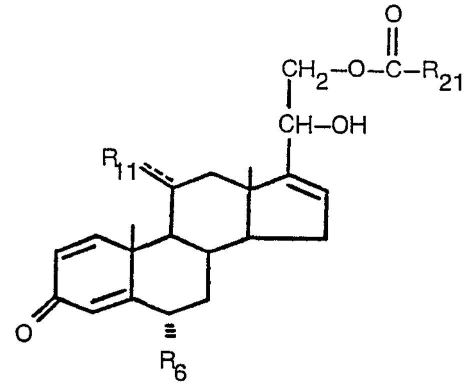

II

wherein $R_6$, $R_{11}$ and $R_{21}$ are as defined in claim 1, which comprises reacting a compound as claimed in claim 1 or claim 2, or the product of a process according to claim 3 or claim 4, with hydrochloric acid or hydrobromic acid.

6. A process according to claim 5, wherein the acid is hydrobromic acid.

7. A process for preparing a compound of formula III:

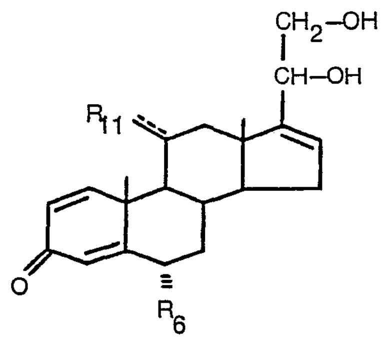

III

wherein $R_6$ and $R_{11}$ are as defined in claim 1, which comprises hydrolysing, with a base, a compound of formula II as defined in claim 5.

8. A process according to claim 7, wherein the base is selected from methoxides, ethoxides, sodium hydroxide, potassium hydroxide, sodium carbonate, potassium carbonate, sodium bicarbonate and potassium bicarbonate.

**Patentansprüche**

1. Verbindung der Formel I

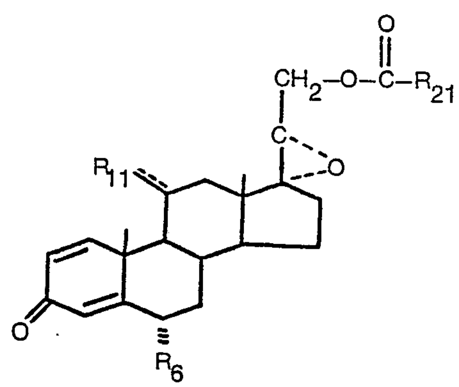

I

worin $R_6$ Wasserstoff, Methyl oder Fluor, $R_{11}$ $\beta$-Hydroxy oder Oxo und $R_{21}$ $C_1$—$C_3$-Alkyl oder Phenyl, das gegebenenfalls mit bis zu drei Substituenten unabhängig ausgewählt aus der Gruppe bestehend aus Fluor, Chlor, Methyl, Methoxy und Nitro substituiert ist, bedeuten.

2. 11$\beta$,21-Dihydroxy-17$\alpha$,20$\alpha$-epoxypregna-1,4-dien-3-on-21-acetat.

3. Verfahren zur Herstellung von Verbindungen gemäss Anspruch 1 oder 2, dadurch gekennzeichnet, dass man ein Olefin der Formel O

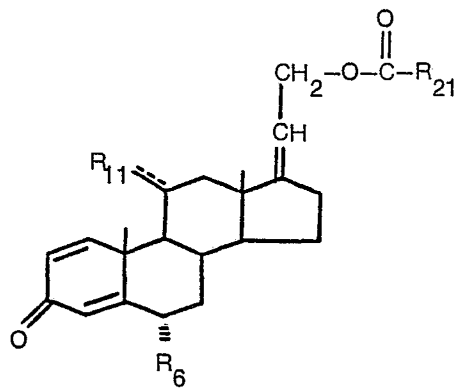

O

worin $R_6$, $R_{11}$ und $R_{21}$ in Anspruch 1 definiert sind, mit mindestens einem molaren Aequivalent einer Persäure oxydiert.

4. Verfahren nach Anspruch 3, dadurch gekennzeichnet, dass die Persäure aus der Gruppe Peressigsäure, Perpropionsäure, Perbenzoesäure und m-Chlorperbenzoesäure ausgewählt ist.

5. Verfahren zur Herstellung einer Verbindung der Formel II

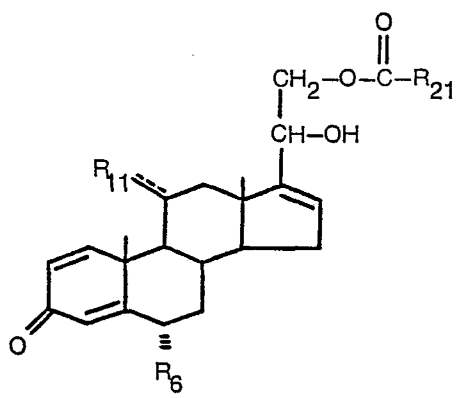

II

worin $R_6$, $R_{11}$ und $R_{21}$ in Anspruch 1 definiert sind, dadurch gekennzeichnet, dass man eine Verbindung gemäss Anspruch 1 oder 2 oder das Verfahrensprodukt gemäss Anspruch 3 oder 4 mit Chlorwasserstoffsäure oder Bromwasserstoffsäure umsetzt.

6. Verfahren nach Anspruch 5, dadurch gekennzeichnet, dass die Säure Bromwasserstoffsäure ist.

7. Verfahren zur Herstellung einer Verbindung der Formel III

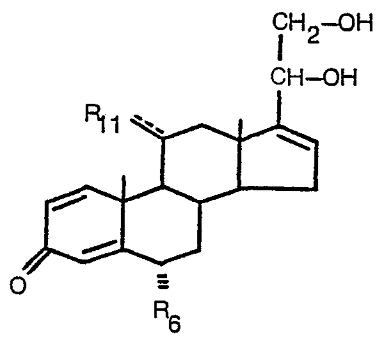

III

worin $R_6$ und $R_{11}$ in Anspruch 1 definiert sind, dadurch gekennzeichnet, dass man eine Verbindung der Formel II gemäss Anspruch 5 mit einer Base hydrolysiert.

8. Verfahren nach Anspruch 7, dadurch gekennzeichnet, dass die Base aus der Gruppe Methoxide, Ethoxide, Natriumhydroxid, Kaliumhydroxid, Natriumcarbonat, Kaliumcarbonat, Natriumbicarbonat und Kaliumbicarbonat ausgewählt ist.

**Revendications**

1. Composé de formule I:

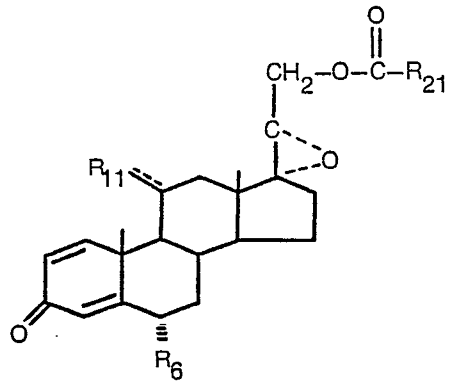

I

dans laquelle $R_6$ est l'hydrogène, le groupe méthyle ou le fluor; $R_{11}$ est un groupe $\beta$-hydroxy ou oxo; et $R_{21}$ est un groupe alkyle en $C_1$ à $C_3$ ou phényle pouvant être substitué avec jusqu'à 3 substituants choisis, indépendamment, entre le fluor, le chlore et les groupes méthyle, méthoxy et nitro.

2. Le 21-acétate de $11\beta$,21-dihydroxy-$17\alpha$,$20\alpha$-époxyprégna-1,4-diène-3-one.

3. Procédé de préparation d'un composé suivant la revendication 1 ou la revendication 2, qui consiste á oxyder une oléfine de formule O:

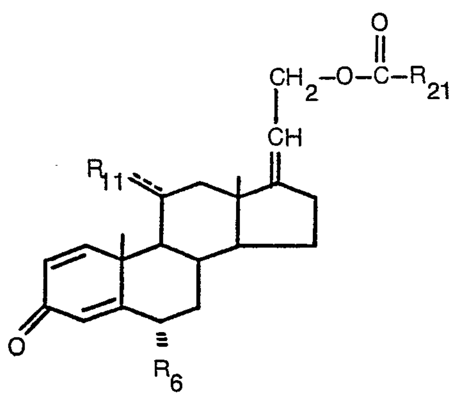

O

dans laquelle $R_6$, $R_{11}$ et $R_{21}$ ont les définitions données dans la revendication 1, avec au moins un équivalent molaire d'un peracide.

4. Procédé suivant la revendication 3, dans lequel le peracide est choisi entre l'acide peracétique, l'acide perpropionique, l'acide perbenzoïque et l'acide *m*-chloroperbenzoïque.

5. Procédé de préparation d'un composé de formule II:

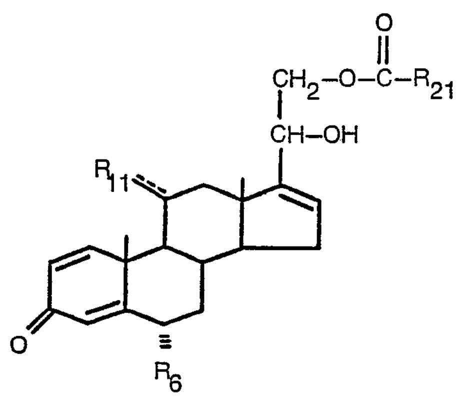

II

dans laquelle $R_6$, $R_{11}$ et $R_{21}$ ont les définitions données dans la revendication 1, qui consiste à faire réagir un composé suivant la revendication 1 ou la revendication 2 ou le produit d'un procédé suivant la revendication 3 ou la revendication 4, avec l'acide chlorhydrique ou l'acide bromhydrique.

6. Procédé suivant la revendication 5, dans lequel l'acide est l'acide bromhydrique.

7. Procédé de préparation d'un composé de formule III:

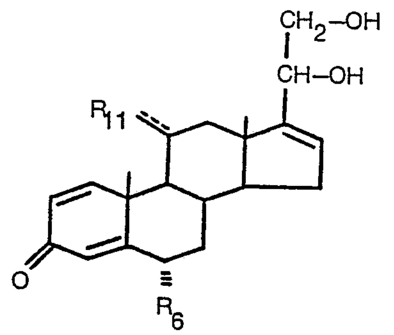

III

dans laquelle $R_6$ et $R_{11}$ ont les définitions données dans la revendication 1, qui consiste à hydrolyser, avec une base, un composé de formule II comme défini dans la revendication 5.

8. Procédé suivant la revendication 7, dans lequel la base est choisie entre des méthylates, des éthylates, l'hydroxyde de sodium, l'hydroxyde de potassium, le carbonate de sodium, le carbonate de potassium, le bicarbonate de sodium et le bicarbonate de potassium.